# EUROPÄISCHE PATENTSCHRIFT

(11) **EP 2 666 420 B1**
(45) Veröffentlichungstag und Bekanntmachung des Hinweises auf die Patenterteilung: **30.08.2017**
(21) Anmeldenummer: 13168282.5
(22) Anmeldetag: 17.05.2013
(51) Int. Cl.: A61B 17/29

(54) **Medizinisches Instrument mit einem Schaft mit einem flexiblen Abschnitt und einem gesteuert krümmbaren Abschnitt**
Medical instrument with a shaft with a flexible section and a controlled bendable section
Instrument médical doté d'une tige ayant une section flexible et une section pouvant être courbée de façon ciblée

(30) Priorität: 23.05.2012 DE 102012208605
(43) Veröffentlichungstag der Anmeldung: 27.11.2013
(73) Patentinhaber: Karl Storz GmbH & Co. KG, 78532 Tuttlingen (DE)
(72) Erfinder: Bacher, Uwe, 78532 Tuttlingen (DE); Zahler, Sabine, 78532 Tuttlingen (DE); Leonhard, Martin, 78532 Tuttlingen (DE)

(56) Entgegenhaltungen:
- WO-A1-2012/106187
- US-A1- 2008 188 868
- US-A1- 2009 054 733
- US-A1- 2009 069 842
- US-A1- 2009 247 994
- US-A1- 2010 030 029
- US-A1- 2010 286 480

## Beschreibung

Die vorliegende Erfindung ist auf ein medizinisches Instrument mit einem Schaft mit einem ungesteuert flexiblen Abschnitt und einem gesteuert krümmbaren Abschnitt gerichtet.

In der mikroinvasiven Chirurgie, insbesondere in der endoluminalen und in der transluminalen mikroinvasiven Chirurgie, werden medizinische Instrumente mit langen, dünnen, flexiblen Schäften verwendet, die in Speiseröhre, Magen und Darm eingeführt werden können. Das distale Ende des flexiblen Schafts kann gesteuert krümmbar sein, um innerhalb eines Hohlraums, beispielsweise ein Schneide- oder Greifwerkzeug beliebig positionieren zu können.

In WO 2012/106187 A1 (Stand der Technik unter Artikel 54(3) EPÜ) ist eine lösbare Kupplung zwischen einer Handhabe und einem Katheter mit einem flexiblen Schaft, einem Werkzeug und einem krümmbaren Steuerabschnitt beschrieben. Handhabe und Katheter können insbesondere durch eine rotatorische Relativbewegung miteinander lösbar gekoppelt werden, wobei je ein Kopf an einem Kopplungsbauglied des Katheters in eine Ausnehmung an einem Kopplungsbauglied der Handhabe eingreift.

In US2009/0054733 A1 ist ein endoskopisch chirurgisches Instrument für endoskopische Eingriffe durch den Gastrointestinaltrakt beschrieben. Das Instrument umfasst einen Handhabungsbereich, einen länglichen Mittelabschnitt, einen gelenkigen Abschnitt und einen Werkzeugabschnitt. Der Werkzeugabschnitt und ein "Werkzeugeinsatz" ("tool insert"), der an einen Bowdenzug erinnert, sind zusammen austauschbar. Der Oberbegriff des Anspruchs 1 basiert auf diesem Dokument. In US 2010/0286480 A1 ist ein chirurgisches Instrument mit einer wiederverwendbaren Handhabe und einer Schaftanordnung, die von der Handhabe trennbar ist, beschrieben. Schaftanordnung und Handhabe sind durch vier Nocken an einem Fassungsring an der Schaftanordnung, die in korrespondierende L-förmige Schlitze 372 an der Handhabe eingreifen, verbindbar.

In US 2008/0188868 A1 ist ein direkt gesteuertes Endoskopiesystem beschrieben. Mittels einer Handhabe, die um zwei orthogonale Achsen schwenk- bzw. rotierbar ist, ist ein gelenkiger distaler Abschnitt steuerbar. Enden von Steuerkabeln sind in Nuten oder Schlitzen in Stiften angeordnet.

Eine Aufgabe der vorliegenden Erfindung besteht darin, ein verbessertes medizinisches Instrument mit einem Schaft mit einem krümmbaren Abschnitt und einem ungesteuert flexiblen Abschnitt, einen Schaft mit einem gesteuert krümmbaren Abschnitt und einem ungesteuert flexiblen Abschnitt und eine Handhabungseinrichtung für ein medizinisches Instrument zu schaffen.

Diese Aufgabe wird durch die Gegenstände der unabhängigen Ansprüche gelöst.

Weiterbildungen sind in den abhängigen Ansprüchen angegeben.

Ein Schaft für ein medizinisches Instrument umfasst ein Werkzeug oder eine Kupplung für ein Werkzeug, einen gesteuert krümmbaren Abschnitt oder ein Gelenk, einen ungesteuert flexiblen Abschnitt und eine Schaftkupplungseinrichtung zur lösbaren mechanischen Verbindung des Schafts mit einer Handhabungseinrichtung.

Der gesteuert krümmbare Abschnitt weist eine steuerbare Krümmung auf. Die Krümmung des flexiblen Abschnitts ist nicht steuerbar. Der gesteuert krümmbare Abschnitt oder das Gelenk ist insbesondere an oder nahe dem distalen Ende des Schafts angeordnet. Die Krümmung des gesteuert krümmbaren Abschnitts bzw. die Winkelposition des Gelenks ist insbesondere mittels einer Handhabungseinrichtung, die mit der Schaftkupplungseinrichtung mechanisch verbunden ist, steuerbar. Der ungesteuert flexible Abschnitt des Schafts ist insbesondere proximal des gesteuert krümmbaren Abschnitts bzw. des Gelenks angeordnet und erstreckt sich über einen großen Teil der Gesamtlänge des Schafts. Der Schaft und seine Eigenschaften und Merkmale sind insbesondere für eine Verwendung in der endoluminalen und in der transluminalen mikroinvasiven Chirurgie vorgesehen und ausgebildet.

Ein Abschnitt eines Schafts ist flexibel, wenn er zumindest bis zu einer maximalen Krümmung bzw. einem minimalen Krümmungsradius reversibel krümmbar und insbesondere vollständig elastisch oder zumindest teilweise elastisch krümmbar ist. Die elastischen Eigenschaften des ungesteuert flexiblen Abschnitts, seine maximale Krümmung und sein minimaler Krümmungsradius sind an die vorgesehene Verwendung des Schafts, insbesondere an die Eigenschaften des Hohlorgans, in dem der Schaft eingesetzt werden soll, angepasst.

Die Schaftkupplungseinrichtung ist insbesondere am proximalen Ende des Schafts angeordnet oder bildet das proximale Ende des Schafts. Die Schaftkupplungseinrichtung ist insbesondere zur starren mechanischen Verbindung des proximalen Endes des Schafts mit einer Handhabungseinrichtung ausgebildet.

Die Schaftkupplung am Schaft ermöglicht eine wiederholte zerstörungsfreie Trennung und eine nachfolgende mechanische Verbindung des Schafts mit einer Handhabungseinrichtung. Deshalb kann beispielsweise ein beschädigter Schaft ersetzt oder repariert werden, während die Handhabungseinrichtung weiter verwendet wird. Entsprechend kann eine beschädigte Handhabungseinrichtung ersetzt oder repariert werden, während der Schaft weiter verwendet wird. Ferner können unterschiedliche Schäfte für unterschiedliche Anwendungen und/oder mit unterschiedlichen Längen, Querschnitten oder anderen Eigenschaften mit der gleichen Handhabungseinrichtung kombiniert werden. Ferner können mit einem Schaft unterschiedliche Handhabungseinrichtungen kombiniert werden, beispielsweise für unterschiedliche Händigkeit oder unterschiedliche Größen der die Handhabungseinrichtung führenden Hand medizinischen Personals. Ferner können sowohl Schaft als auch Handhabungseinrichtung unabhängig voneinander weiterentwickelt werden. Deshalb kann eine einmedizinische Institution von den Weiterentwicklungen profitieren, indem sie lediglich den weiterentwickelten Schaft oder lediglich die weiterentwickelte Handhabungseinrichtung neu erwirbt und den jeweils anderen Bestandteil aus ihrem Bestand weiterverwendet.

Die Modularität des medizinischen Instruments aus Schaft und Handhabungseinrichtung ermöglicht somit unter anderem eine vielfältigere Verwendung bzw. eine Anpassung an eine größere Vielfalt von Verwendungen und/oder das Vorhalten einer kleineren Stückzahl von Schäften und Handhabungseinrichtungen mit entsprechend geringeren Investitionskosten und entsprechend geringerem logistischem Aufwand.

Ein Schaft, wie er hier beschrieben ist, umfasst insbesondere ferner eine Übertragungseinrichtung zum Übertragen zumindest entweder einer Kraft oder eines Drehmoments zwischen dem proximalen Ende des Schafts und dem gesteuert krümmbaren Abschnitt oder dem Gelenk.

Die Übertragungseinrichtung ist insbesondere zum mechanischen Übertragen einer Druck- und/oder Zugkraft und/oder eines Drehmoments ausgebildet. Um die Flexibilität des insbesondere proximal des gesteuert krümmbaren Abschnitts angeordneten ungesteuert flexiblen Abschnitts nicht zu beeinträchtigen, ist auch die Übertragungseinrichtung selbst biegeflexibel. Die Übertragungseinrichtung erstreckt sich vom proximalen Ende des Schafts bis zu dem gesteuert krümmbaren Abschnitt oder dem Gelenk.

Bei einem Schaft mit einer Übertragungseinrichtung, wie er hier beschrieben ist, umfasst die Übertragungseinrichtung insbesondere ein Zugseil oder einen Zugdraht.

Die Übertragungseinrichtung kann zwei, drei, vier oder mehr nebeneinander angeordnete Zugseile oder Zugdrähte, die sich jeweils vom proximalen Ende des Schafts bis zum gesteuert krümmbaren Abschnitt des Schafts erstrecken, umfassen. Das oder die Zugseile sind insbesondere jeweils ausschließlich zur Übertragung von Zugkräften geeignet. Alternativ oder zusätzlich kann die Übertragungseinrichtung ein oder mehrere Bauteile umfassen, die für eine Übertragung von Druck- bzw. Schubkräften oder sowohl für eine Übertragung von Zugkräften als auch für eine Übertragung von Druck- bzw. Schubkräften ausgebildet sind.

Ein Zugseil oder ein Zugdraht kann bei einem verhältnismäßig geringen Querschnitt und damit auch bei verhältnismäßig großer mechanischer Flexibilität für die Übertragung einer großen Zugkraft ausgebildet sein. Mittels eines Zugseils oder eines Zugdrahts kann die Krümmung des gesteuert krümmbaren Abschnitts oder die Abwinklung des Gelenks in einer Ebene gesteuert werden. Dabei bewirkt beispielsweise eine Feder oder ein anderes elastisches Element eine Rückstellung des gesteuert krümmbaren Abschnitts oder des Gelenks in eine vorbestimmte Position, wenn das proximale Ende des Zugseils oder des Zugdrahts freigegeben wird. Mit zwei, drei oder mehr Zugseilen oder Zugdrähten ist eine Rückstellung auch ohne ein elastisches Element und/oder eine Krümmung des gesteuert krümmbaren Abschnitts oder eine Abwinklung des Gelenks in zwei zueinander senkrechten Richtungen möglich Eine Krümmung des gesteuert krümmbaren Abschnitts oder eine Abwinklung des Gelenks in zwei zueinander senkrechten Richtungen bewirkt, dass das distale Ende des Schafts nicht nur auf einem Bogen (im Fall des Gelenks: Kreisbogen), sondern auf einem Ausschnitt einer kuppelförmig gekrümmten Fläche (im Fall des Gelenks: insbesondere Kugeloberfläche) bewegt werden kann.

Ein Schaft mit einer Übertragungseinrichtung, wie er hier beschrieben ist, umfasst insbesondere ferner eine Übertragungskupplungseinrichtung am proximalen Ende des Schafts zum lösbaren mechanischen Koppeln mit einer korrespondierenden Übertragungskupplungseinrichtung an einer Handhabungseinrichtung.

Mittels der Übertragungskupplungseinrichtung am Schaft und einer korrespondierenden Übertragungskupplungseinrichtung an einer Handhabungseinrichtung sind die Übertragungseinrichtung des Schafts und eine Betätigungseinrichtung an der Handhabungseinrichtung mechanisch koppelbar. Dadurch ist die Krümmung des gesteuert krümmbaren Abschnitts oder die Abwinklung des Gelenks durch manuelle Betätigung der Betätigungseinrichtung an der Handhabungseinrichtung steuerbar.

Bei einem Schaft mit einer Übertragungskupplungseinrichtung, wie er hier beschrieben ist, ist die Übertragungskupplungseinrichtung insbesondere für eine Rastverbindung mit einer korrespondierenden Übertragungskupplungseinrichtung an einer Handhabungseinrichtung ausgebildet.

Für eine Rastverbindung weist die Übertragungskupplungseinrichtung insbesondere einen elastischen Abschnitt und/oder einen rampenförmigen Abschnitt auf. Die Rastverbindung ermöglicht ein selbsttätiges bzw. ohne Weiteres erfolgendes formschlüssiges, mechanisches Verbinden der Übertragungskupplungseinrichtung mit einer korrespondierenden Übertragungskupplungseinrichtung einer Handhabungseinrichtung. Dadurch kann die Kombination eines Schafts und einer Handhabungseinrichtung zu einem medizinischen Instrument vereinfacht und medizinisches Personal unterstützt werden.

Bei einem Schaft mit einer Übertragungskupplungseinrichtung, wie er hier beschrieben ist, weist die Übertragungskupplungseinrichtung an ihrem proximalen Ende insbesondere einen konkaven Abschnitt auf.

Der konkave Abschnitt weist insbesondere eine Gestalt auf, die zu einem konvexen Abschnitt einer korrespondierenden Übertragungskupplungseinrichtung an einer Handhabungseinrichtung korrespondiert. Ein konkaver Abschnitt ist ein Abschnitt mit einer Vertiefung bzw. einer Ausnehmung. Ein konvexer Abschnitt ist ein vorragender bzw. vorstehender Abschnitt. Insbesondere ist der konkave Abschnitt zur Aufnahme eines kugelförmigen Abschnitts an einer korrespondierenden Übertragungskupplungseinrichtung ausgebildet.

Alternativ weist bei einem Schaft mit einer Übertragungskupplungseinrichtung, wie er hier beschrieben ist, die Übertragungskupplungseinrichtung an ihrem proximalen Ende einen konvexen Abschnitt auf. Der konvexe Abschnitt weist insbesondere eine zu einem konkaven Abschnitt einer korrespondierenden Übertragungskupplungseinrichtung an einer Handhabungseinrichtung korrespondierende Gestalt auf. Insbesondere weist der konvexe Abschnitt einen kugelflächenförmigen Bereich auf.

Bei einem Schaft mit einer Übertragungskupplungseinrichtung, wie er hier beschrieben ist, sind die Schaftkupplungseinrichtung und die Übertragungskupplungseinrichtung insbesondere so angeordnet und ausgebildet, dass eine mechanische Verbindung zwischen der Schaftkupplungseinrichtung und einer korrespondierenden Kupplungseinrichtung an einer Handhabungseinrichtung und eine mechanische Kopplung der Übertragungskupplungseinrichtung mit einer korrespondierenden Übertragungskupplungseinrichtung an der Handhabungseinrichtung mittels einer Rotation des Schafts relativ zur Handhabungseinrichtung lösbar sind.

Insbesondere sind die starre mechanische Verbindung der Schaftkupplungseinrichtung des Schafts mit der korrespondierenden Kupplungseinrichtung der Handhabungseinrichtung (und damit die starre mechanische Verbindung des proximalen Endes des Schafts mit der Handhabungseinrichtung) und die mechanische Kopplung der Übertragungskupplungseinrichtung des Schafts mit der korrespondierenden Übertragungskupplungseinrichtung der Handhabungseinrichtung (und damit die mechanische Kopplung der Übertragungseinrichtung des Schafts mit einer zugeordneten Betätigungseinrichtung der Handhabungseinrichtung) mittels einer Rotation des Schafts um die Längsachse des proximalen Endes des Schafts lösbar. Dabei werden die mechanische Verbindung zwischen der Schaftkupplungseinrichtung des Schafts und der korrespondierenden Kupplungseinrichtung der Handhabungseinrichtung und die mechanische Kopplung der Übertragungskupplungseinrichtung des Schafts mit der korrespondierenden Übertragungskupplungseinrichtung der Handhabungseinrichtung gleichzeitig oder im Wesentlichen gleichzeitig oder nacheinander bei einer relativen Rotation um einen vorbestimmten Winkel gelöst.

Mit einer einzigen Rotationsbewegung des Schafts relativ zur Handhabungseinrichtung sowohl die mechanische Verbindung zwischen der Schaftkupplungseinrichtung des Schafts und der korrespondierenden Kupplungseinrichtung der Handhabungseinrichtung als auch die mechanische Kopplung der Übertragungskupplungseinrichtung mit der korrespondierenden Übertragungskupplungseinrichtung der Handhabungseinrichtung lösen zu können, bedeutet eine Vereinfachung der Handhabung beim Zerlegen eines medizinischen Instruments in den Schaft und die Handhabungseinrichtung. Dies reduziert die zum Zerlegen erforderliche Zeitdauer und das Risiko einer Beschädigung aufgrund eines Fehlers beim Zerlegen.

Ein Schaft, wie er hier beschrieben ist, umfasst insbesondere mehrere Übertragungskupplungseinrichtungen. Jede einzelne Übertragungskupplungseinrichtung weist insbesondere die oben beschriebenen Merkmale auf. Jede einzelne Übertragungskupplungseinrichtung ist insbesondere korrespondierend zu und zur Kopplung mit einer Übertragungskupplungseinrichtung einer Handhabungseinrichtung vorgesehen und angeordnet.

Bei einem Schaft, wie er hier beschrieben ist, sind am proximalen Ende des Schafts insbesondere mehrere Übertragungskupplungseinrichtungen zur Steuerung des gesteuert krümmbaren Abschnitts vorgesehen.

Die Übertragungskupplungseinrichtungen sind insbesondere gleich bzw. entsprechen einander hinsichtlich ihrer Gestalt und ihren sonstigen Eigenschaften. Jede Übertragungskupplungseinrichtung ist insbesondere mit einem zugeordneten Zugseil oder einem zugeordneten Zugdraht verbunden.

Ein Schaft, wie er hier beschrieben ist, umfasst insbesondere ferner eine weitere Übertragungseinrichtung zum Übertragen zumindest entweder einer Kraft oder eines Drehmoments zwischen dem proximalen Ende des Schafts und einem Werkzeug am distalen Ende des Schafts und eine weiteren Übertragungskupplungseinrichtung zum mechanischen Koppeln der Übertragungseinrichtung mit einer korrespondierenden Übertragungskupplungseinrichtung an einer Handhabungseinrichtung, wobei die weitere Übertragungseinrichtung außerhalb der Schaftkupplungseinrichtung angeordnet ist.

Insbesondere ist die weitere Übertragungseinrichtung außerhalb der Schaftkupplungseinrichtung und beabstandet von der Schaftkupplungseinrichtung angeordnet. Diese Anordnung der weiteren Übertragungskupplungseinrichtung kann ein separates manuelles Koppeln und Entkoppeln der weiteren Übertragungseinrichtung und einer zur weiteren Übertragungskupplungseinrichtung korrespondierenden weiteren Betätigungseinrichtung an einer Handhabungseinrichtung ermöglichen.

Eine Handhabungseinrichtung für ein medizinisches Instrument umfasst eine Schaftkupplungseinrichtung zur lösbaren mechanischen Verbindung der Handhabungseinrichtung mit einem Schaft mit einem gesteuert krümmbaren Abschnitt oder einem Gelenk und einem ungesteuert flexiblen Abschnitt, eine erste Betätigungseinrichtung zum Steuern eines Werkzeugs am distalen Ende des Schafts, eine zweite Betätigungseinrichtung zum manuellen Steuern der Krümmung des gesteuert krümmbaren Abschnitts oder des Gelenks des Schafts und eine Übertragungskupplungseinrichtung zum lösbaren mechanischen Koppeln der zweiten Betätigungseinrichtung mit einer Übertragungseinrichtung zum Übertragen zumindest entweder einer Kraft oder eines Drehmoments zu dem gesteuert krümmbaren Abschnitt oder Gelenk des Schafts.

Die Handhabungseinrichtung ist insbesondere vorgesehen, um mit einem Schaft, wie er hier beschrieben ist, ein medizinisches Instrument zu bilden. Die Schaftkupplungseinrichtung ist insbesondere am distalen Ende der Handhabungseinrichtung und insbesondere zur lösbaren starren mechanischen Verbindung der Handhabungseinrichtung mit dem proximalen Ende eines Schafts ausgebildet. Die Übertragungskupplungseinrichtung ist insbesondere ebenfalls am distalen Ende der Handhabungseinrichtung angeordnet.

Bei einer Handhabungseinrichtung, wie sie hier beschrieben ist, ist die Übertragungskupplungseinrichtung insbesondere für eine Rastverbindung mit einer korrespondierenden Übertragungskupplungseinrichtung an einem mit der Handhabungseinrichtung verbundenen Schaft ausgebildet.

Dazu weist die Übertragungskupplungseinrichtung der Handhabungseinrichtung insbesondere einen elastischen Abschnitt und/oder einen rampenartigen Abschnitt auf.

Bei einer Handhabungseinrichtung, wie sie hier beschrieben ist, weist die Übertragungskupplungseinrichtung insbesondere an ihrem distalen Ende einen konvexen Abschnitt auf.

Der konvexe Abschnitt weist insbesondere eine zu einem konkaven Abschnitt einer Übertragungskupplungseinrichtung an einem mit der Handhabungseinrichtung verbundenen Schaft korrespondierende Gestalt auf. Insbesondere weist der konvexe Abschnitt kugelflächenförmige Oberflächenbereiche auf.

Alterativ weist bei einer Handhabungseinrichtung, wie sie hier beschrieben ist, die Übertragungskupplungseinrichtung insbesondere an ihrem proximalen Ende einen konkaven Abschnitt auf. Der konkave Abschnitt weist insbesondere eine zu einem konvexen Abschnitt einer Übertragungskupplungseinrichtung an einem mit der Handhabungseinrichtung verbundenen flexiblen Schaft korrespondierende Gestalt auf.

Bei einer Handhabungseinrichtung, wie sie hier beschrieben ist, ist die zweite Betätigungseinrichtung insbesondere um eine Achse rotierbar oder schwenkbar, wobei die Übertragungskupplungseinrichtung mittels eines Pleuels mit der zweiten Betätigungseinrichtung mechanisch gekoppelt ist. Die Achse, um die die zweite Betätigungseinrichtung rotierbar oder schwenkbar ist, ist insbesondere senkrecht zur Längsachse des proximalen Endes eines mit der Handhabungseinrichtung mechanisch verbundenen Schafts.

Bei einer Handhabungseinrichtung, wie sie hier beschrieben ist, sind die Schaftkupplungseinrichtung und die Übertragungskupplungseinrichtung insbesondere so angeordnet und ausgebildet, dass eine mechanische Verbindung zwischen der Schaftkupplungseinrichtung und einer korrespondierenden Kupplungseinrichtung an einem Schaft und eine mechanische Kopplung der Übertragungskupplungseinrichtung mit einer korrespondierenden Übertragungskupplungseinrichtung an dem Schaft mittels einer Rotation der Handhabungseinrichtung relativ zum Schaft lösbar sind.

Bei einem Schaft, wie er hier beschrieben ist, und bei einer Handhabungseinrichtung, wie sie hier beschrieben ist, sind die Schaftkupplungseinrichtungen insbesondere als Bajonettkupplungen ausgebildet. Zum Verriegeln der Kupplungseinrichtung kann eine Verriegelungseinrichtung vorgesehen sein. Eine solche Verriegelungseinrichtung kann einen Riegel umfassen, der parallel oder im Wesentlichen parallel zur Längsachse des proximalen Endes des Schafts bewegbar ist.

Eine Handhabungseinrichtung, wie sie hier beschrieben ist, umfasst insbesondere mehrere Übertragungskupplungseinrichtungen, wobei jede Übertragungskupplungseinrichtung die hier beschriebenen Eigenschaften einer Übertragungskupplungseinrichtung aufweisen kann.

Bei einem Schaft, wie er hier beschrieben ist, oder bei einer Handhabungseinrichtung, wie sie hier beschrieben ist, sind insbesondere mehrere Übertragungskupplungseinrichtungen symmetrisch bezüglich der Längsachse des Schafts angeordnet.

Bei der Handhabungseinrichtung, wie er hier beschrieben ist, sind die Übertragungskupplungseinrichtungen insbesondere symmetrisch zur Längsachse eines proximalen Endes eines mit der Handhabungseinrichtung mechanisch verbundenen Schafts angeordnet.

Eine symmetrische Anordnung mehrerer Übertragungskupplungseinrichtungen bezüglich einer Symmetrieachse liegt dann vor, wenn die Orte und die Orientierungen der Übertragungskupplungseinrichtungen durch Rotation um einen ganzzahligen Bruchteil von 360° um die Symmetrieachse auseinander hervorgehen bzw. ineinander übergeführt werden.

Ein medizinisches Instrument umfasst einen Schaft, wie er hier beschrieben ist, und eine Handhabungseinrichtung, wie sie hier beschrieben ist.

Eigenschaften, Merkmale und Vorteile der Handhabungseinrichtung und des medizinischen Instruments entsprechen weitgehend oder vollständig den oben für den Schaft beschriebenen.

### Kurzbeschreibung der Figuren

Nachfolgend werden Ausführungsformen anhand der beigefügten Figuren näher erläutert. Es zeigen:
- Figur 1: eine schematische Darstellung eines medizinischen Instruments;
- Figur 2: eine weitere schematische Darstellung eines Teils des medizinischen Instruments aus Figur 1;
- Figur 3: eine schematische axonometrische Darstellung eines distalen Endes einer Handhabungseinrichtung;
- Figur 4: eine schematische axonometrische Darstellung eines proximalen Endes eines Schafts;
- Figur 5: eine weitere schematische Darstellung des distalen Endes aus Figur 3 und des proximalen Endes aus Figur 4;
- Figur 6: eine schematische Schnittdarstellung des distalen Endes aus den Figuren 3 und 5 und des proximalen Endes aus den Figuren 4 und 5;
- Figur 7: eine schematische Darstellung einer Übertragungskupplungseinrichtung;
- Figur 8: eine weitere schematische Darstellung der Übertragungskupplungseinrichtung aus Figur 7;
- Figur 9: eine schematische axonometrische Darstellung der Übertragungskupplungseinrichtung aus den Figuren 7 und 8;
- Figur 10: eine schematische Darstellung eines Kopplungsvorgangs;
- Figur 11: eine schematische Darstellung eines Entkopplungsvorgangs;
- Figur 12: eine schematische Darstellung eines weiteren Kopplungsvorgangs.

### Beschreibung der Ausführungsformen

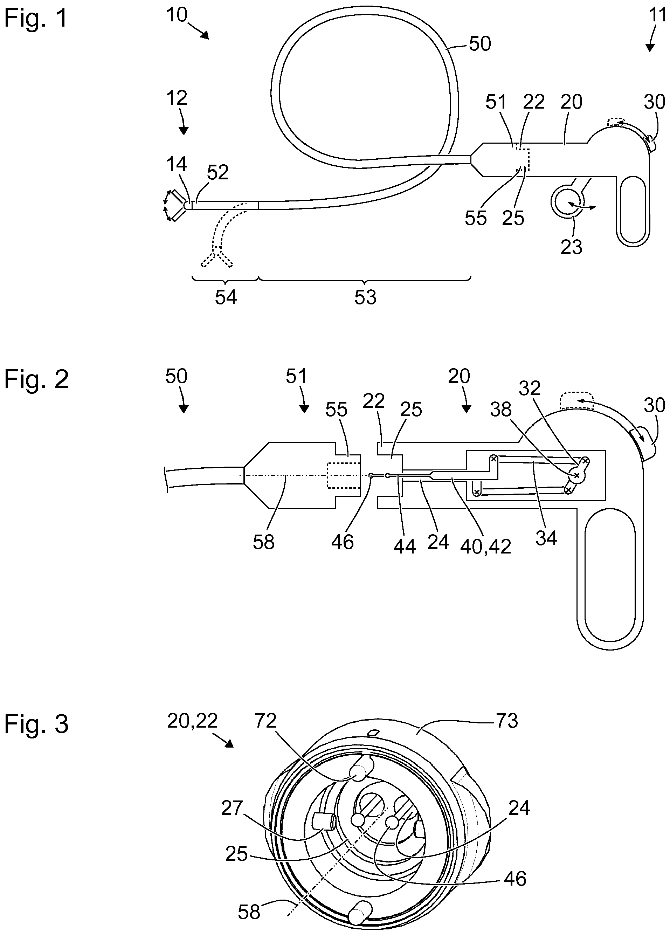

Figur 1 zeigt eine schematische Darstellung eines medizinischen Instruments 10 mit einem proximalen Ende 11 und einem distalen Ende 12. Das medizinische Instrument 10 ist insbesondere für eine Verwendung bei mikroinvasiven chirurgischen Eingriffen oder bei anderen mikroinvasiven diagnostischen, therapeutischen und/oder chirurgischen Verfahren vorgesehen und ausgebildet. Das medizinische Instrument 10 weist am distalen Ende 12 ein Werkzeug 14 auf, das in Figur 1 beispielhaft als Greif- oder Schneidewerkzeug mit zwei schwenkbaren Maulteilen angedeutet ist. Am proximalen Ende 11 weist das medizinische Instrument 10 eine Handhabungseinrichtung 20 auf. Das distale Ende 22 der Handhabungseinrichtung 20 ist durch einen Schaft 50 mit dem Werkzeug 14 verbunden.

Die Handhabungseinrichtung 20 umfasst ein bewegbares, insbesondere ein schwenkbares Griffteil 23, das mittels einer in Figur 1 nicht dargestellten Übertragungseinrichtung im Schaft 50 mit den Maulteilen des Werkzeugs 14 mechanisch gekoppelt ist. Das schwenkbare Griffteil 23 ist eine Betätigungseinrichtung für das Werkzeug 14. Durch Pfeile am bewegbaren Griffteil 23 und an den Maulteilten des Werkzeugs 14 ist angedeutet, dass eine Bewegung des bewegbaren Griffteils 23 mit einer Bewegung der Maulteile des Werkzeugs 14 einhergeht.

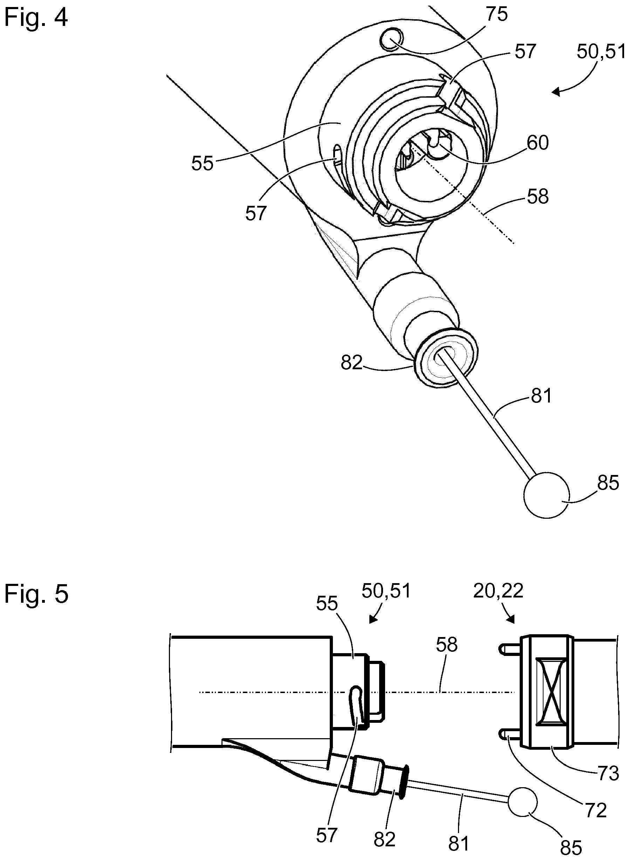

Das proximale Ende 51 des Schafts 50 ist mit der Handhabungseinrichtung 20 mechanisch starr, aber lösbar verbunden. Insbesondere greift ein konvexer Bereich 55 am proximalen Ende 51 des Schafts 50 in eine Ausnehmung 25 am distalen Ende 22 der Handhabungseinrichtung 20 ein. Beispiele für Details der starren und lösbaren mechanischen Verbindung des distalen Endes 22 der Handhabungseinrichtung 20 mit dem proximalen Ende 51 des Schafts 50 sind unten mit Bezug auf die Figuren 4 bis 6 beschrieben. Das distale Ende 52 des Schafts 50 ist dauerhaft bzw. nicht zerstörungsfrei lösbar oder alternativ zerstörungsfrei lösbar mit dem Werkzeug 14 mechanisch verbunden.

Der Schaft 50 weist einen ungesteuert flexiblen Abschnitt 53 und einen gesteuert krümmbaren Abschnitt 54 auf. Die Eigenschaften des ungesteuert flexiblen Abschnitts 53 des Schafts 50, insbesondere seine elastischen (und optional im geringeren Maße auch plastischen) Eigenschaften, der minimale erreichbare Krümmungsradius, sein Querschnitt und seine Oberflächeneigenschaften, sind an die vorgesehene Anwendung des medizinischen Instruments 10 angepasst, um beispielsweise ein Einführen in die Speiseröhre, den Magen oder den Darm eines Patienten zu ermöglichen.

Der gesteuert krümmbare Abschnitt 54 des Schafts 50 ist mittels einer weiteren, in Figur 1 ebenfalls nicht dargestellten Übertragungseinrichtung im Inneren des Schafts 50 mit einer Betätigungseinrichtung 30 an der Handhabungseinrichtung 20 mechanisch gekoppelt. In Figur 1 sind jeweils einmal in durchgezogenen und einmal in gestrichelten Linien die Betätigungseinrichtung 30 in zwei verschiedenen Positionen und der gesteuert krümmbare Abschnitt 54 des Schafts 50 in zwei verschiedenen räumlichen Konfigurationen bzw. mit zwei verschiedenen Krümmungen dargestellt. Die beiden in Figur 1 dargestellten Positionen der Betätigungseinrichtung 30 und die beiden in Figur 1 dargestellten Krümmungen des gesteuert krümmbaren Abschnitts 54 des Schafts 50 stellen beispielsweise zwei extreme Positionen bzw. zwei extreme Krümmungszustände dar. Insbesondere entsprechen einander die in durchgezogener Linie dargestellte Position der Betätigungseinrichtung 30 und der in durchgezogenen Linien dargestellte Krümmungszustand des gesteuert krümmbaren Abschnitts 54. Insbesondere entsprechen einander die in gestrichelter Linie dargestellte Position der Betätigungseinrichtung 30 und der in gestrichelten Linien dargestellte Krümmungszustand des gesteuert krümmbaren Abschnitts 54 des Schafts 50. Medizinisches Personal kann durch manuelles Betätigen der Betätigungseinrichtung 30 bzw. durch manuelles Verschieben der Betätigungseinrichtung 30 an der Handhabungseinrichtung 20 den Krümmungszustand des gesteuert krümmbaren Abschnitts 54 verändern und einstellen.

Figur 2 zeigt eine vergrößerte schematische Darstellung der Handhabungseinrichtung 20 und des proximalen Endes 51 des Schafts 50 aus Figur 1. Die Handhabungseinrichtung 20 ist in Figur 2 teilweise aufgeschnitten dargestellt, um im Inneren der Handhabungseinrichtung 20 angeordnete Einrichtungen und Merkmale zu zeigen.

Die Betätigungseinrichtung 30 ist mit einer Kurbel 32 derart mechanisch gekoppelt, dass ein manuelles Bewegen der Betätigungseinrichtung 30 mit einem Schwenken der Kurbel 32 um ihre Schwenkachse 38 einhergeht. Insbesondere ist die Betätigungseinrichtung 30 entlang eines Kreisbogens, dessen Mittelpunkt auf der Schwenkachse 38 liegt, verschiebbar und mit der Kurbel 32 starr verbunden.

In zwei Führungskanälen 24 in der Handhabungseinrichtung 20 sind je eine Übertragungskupplungseinrichtung 40 angeordnet. Einer der beiden Führungskanäle 24 ist in Figur 2 angeschnitten, der zweite Führungskanal liegt bei dem dargestellten Beispiel in Deckung hinter der Zeichenebene. Die Führungskanäle 24 und die Übertragungskupplungseinrichtungen 40 sind derart ausgebildet, dass die Übertragungskupplungseinrichtungen 40 in den Führungskanälen 24 in einer vorbestimmten Richtung linear verschiebbar und in beiden Richtungen senkrecht zu der vorbestimmten Richtung spiel- und reibungsarm geführt sind.

Eine Übertragungskupplungseinrichtung 40 weist jeweils einen im Führungskanal 24 geführten Abschnitt 42 auf, dessen Querschnitt an den Querschnitt des Führungskanals angepasst ist. Das proximale Ende jeder Übertragungskupplungseinrichtung 40 ist über ein zugeordnetes Pleuel 34 mit einem Ende der Kurbel 32 gelenkig verbunden. Die beiden Enden der Kurbel 32, die über je ein Pleuel 34 mit einem proximalen Ende einer Übertragungskupplungseinrichtung 40 gekoppelt sind, sind bei dem dargestellten Beispiel bezogen auf die Schwenkachse 38 der Kurbel 32 von der Schwenkachse 38 in entgegengesetzten Richtungen beabstandet. Eine Bewegung der Betätigungseinrichtung 30 geht aufgrund der (insbesondere starren) Kopplung der Betätigungseinrichtung 30 mit der Kurbel 32 und vermittels der Pleuel 34 mit linearen Verschiebungen der Übertragungskupplungseinrichtungen 40 in zwei entgegengesetzte Richtungen einher.

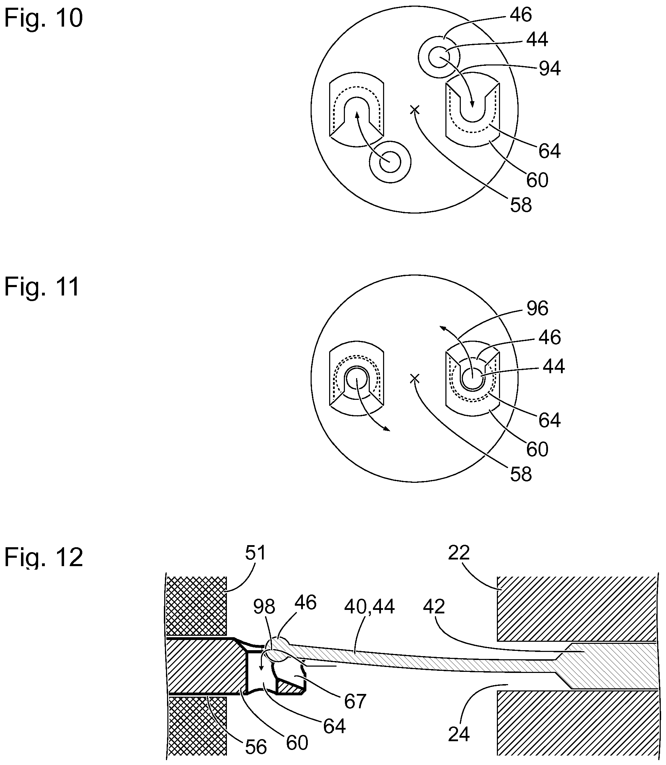

Jede Übertragungskupplungseinrichtung 40 weist distal des im Führungskanal 24 geführten Abschnitts 42 einen elastischen Abschnitt 44 und am distalen Ende des elastischen Abschnitts 44 eine Kugel 46 auf. Die Elastizität der elastischen Abschnitte 44 der Übertragungskupplungseinrichtungen 40 beruht jeweils insbesondere auf einem gegenüber dem im Führungskanal 24 geführten Abschnitt 42 deutlich verringerten Querschnitt. Die Querschnitte der elastischen Abschnitte 44 der Übertragungskupplungseinrichtungen 40 sind beispielsweise kreisförmig, quadratisch oder rechteckig mit langen Seiten in Richtung senkrecht zur Zeichenebene der Figur 2. Die Elastizität der elastischen Abschnitte 44 der Übertragungskupplungseinrichtungen 40 ermöglicht eine Auslenkung der Kugeln 46 an den distalen Enden der elastischen Abschnitte 44 aus den in Figur 2 gezeigten Ruhepositionen. Diese Auslenkung ist unten mit Bezug auf Figur 12 dargestellt.

Das proximale Ende 51 des Schafts 50 weist eine Längsachse 58 auf. Der konvexe Bereich 55 am proximalen Ende 51 des Schafts 50 ist im Wesentlichen rotationssymmetrisch zur Längsachse 58, insbesondere abschnittsweise kreiszylindrisch. Die Ausnehmung 25 am distalen Ende 22 der Handhabungseinrichtung 20 ist ebenfalls im Wesentlichen rotationssymmetrisch zur Längsachse 58 des proximalen Endes 51 des flexiblen Schafts 50, wenn das proximale Ende 51 relativ zur Handhabungseinrichtung 20 in der in Figur 2 gezeigten Weise ausgerichtet ist.

Ausgehend von der in Figur 2 gezeigten Anordnung und Ausrichtung des proximalen Endes 51 des Schafts 50 relativ zum distalen Ende 22 der Handhabungseinrichtung 20 können beide zusammengefügt werden. Dazu werden das proximale Ende 51 des Schafts 50 parallel zur Längsachse 58 auf das distale Ende 22 der Handhabungseinrichtung 20 zu bewegt und der konvexe Bereich 55 in die Ausnehmung 25 eingeführt, wie in Figur 1 angedeutet. Die Querschnitte der Ausnehmung 25 am distalen Ende 22 der Handhabungseinrichtung 20 und des konvexen Bereichs 55 am proximalen Ende 51 des Schafts 50 sind so einander angepasst, dass bei der in Figur 1 gezeigten Konfiguration der konvexe Bereich 55 am proximalen Ende 51 des Schafts 50 spielarm in der Ausnehmung 25 am distalen Ende 22 der Handhabungseinrichtung 20 geführt ist. Formschluss zwischen dem konvexen Bereich 55 und der Ausnehmung 25 fixiert die Ausrichtung des proximalen Endes 51 des Schafts 50 relativ zum distalen Ende 22 der Handhabungseinrichtung 20. Durch eine nachfolgende Rotationsbewegung des proximalen Endes 51 des Schafts 50 relativ zum distalen Ende 22 der Handhabungseinrichtung 20 kann eine zugfeste Verbindung zwischen beiden in der Art einer Bajonettverbindung hergestellt werden.

Bei der vorgesehenen und in Figur 2 dargestellten relativen Ausrichtung des proximalen Endes 51 des Schafts 50 relativ zum distalen Ende 22 der Handhabungseinrichtung 20 sind die durch die Führungskanäle 24 vorgegebenen Richtungen, in denen die Übertragungskupplungseinrichtungen 40 verschiebbar sind, parallel zur Längsachse 58 angeordnet. Insbesondere sind die Übertragungskupplungseinrichtungen 40 symmetrisch zur Längsachse 58 angeordnet, d. h. die Positionen und Orientierungen der Übertragungskupplungseinrichtungen 40 gehen durch Rotation um die Längsachse 58 um 180 Grad aus einander hervor.

Figur 3 zeigt eine schematische axonometrische Darstellung des distalen Endes 22 einer Handhabungseinrichtung 20, die in einigen Merkmalen und Eigenschaften der Handhabungseinrichtung aus den Figuren 1 und 2 ähnelt. Nachfolgend sind vor allem die Merkmale und Eigenschaften beschrieben, die die anhand der Figuren 1 und 2 dargestellte Handhabungseinrichtung nicht aufweist, oder die in den Figuren 1 und 2 nicht dargestellt sind.

In der Ausnehmung 25 sind an einander gegenüberliegenden Orten zwei Bajonettnasen bzw. Knaggen 27 angeordnet. Die Knaggen 27 sind insbesondere in Bohrungen in der die Ausnehmung 25 umschließenden Wand eingesetzte Stifte kreiszylindrischer Gestalt.

Zwei jeweils im Wesentlichen stiftförmige Riegel 72 sind an zwei gegenüberliegenden Seiten des Rands der Ausnehmung 25 angeordnet. Ein Ring 73 ist starr mit den beiden Riegeln 72 verbunden. Der Ring 73 ist zusammen mit den Riegeln 72 relativ zum distalen Ende 22 der Handhabungseinrichtung 20 in Richtung parallel zur Längsachse 58 verschiebbar. Ein oder mehrere in Figur 3 nicht gezeigte Federn oder andere elastische Elemente halten den Ring 73 und die Riegel 72 in der in Figur 3 gezeigten distalen Position. Manuell kann der Ring 73 zusammen mit den Riegeln 72 gegen die Kraft der Federn oder anderen elastischen Elemente nach proximal verschoben werden.

Figur 4 zeigt eine schematische axonometrische Darstellung des proximalen Endes 51 eines Schafts 50, der in einigen Merkmalen und Eigenschaften dem Schaft aus den Figuren 1 und 2 ähnelt. Nachfolgend sind vor allem die Merkmale und Eigenschaften beschrieben, die der anhand der Figuren 1 und 2 dargestellte Schaft nicht aufweist, oder die in den Figuren 1 und 2 nicht dargestellt sind.

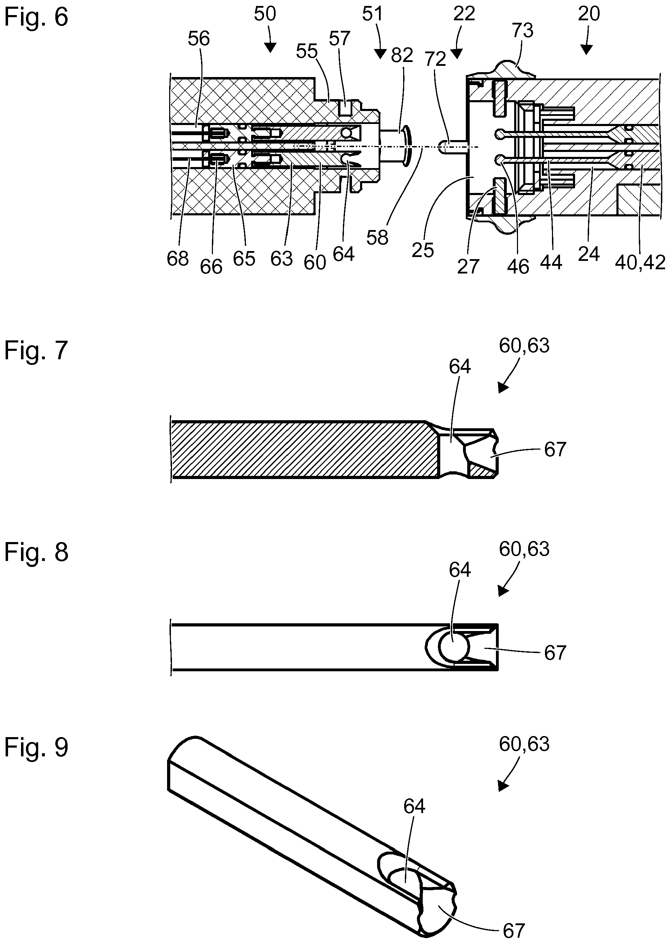

In einer Ausnehmung im konvexen Bereich 55 des proximalen Endes 51 des Schafts 50 sind die proximalen Enden zweier Übertragungskupplungseinrichtungen 60 erkennbar, die und deren Funktion unten mit Bezug auf die Figuren 7 bis 12 näher dargestellt sind. Am äußeren Umfang des konvexen Bereichs 55 sind einander gegenüberliegend angeordnet zwei jeweils L-förmige Nuten 57 vorgesehen. Jede L-förmige Nut 57 weist einen Abschnitt parallel zur Längsachse 58 des proximalen Endes 51 des Schafts 50 und einen weiteren Abschnitt, der sich im Wesentlichen in umfänglicher Richtung erstreckt, auf. Anordnung und Gestalt der in Figur 3 gezeigten Knaggen 27 am distalen Ende 22 der Handhabungseinrichtung 20 einerseits und der L-förmigen Nuten 57 am proximalen Ende 51 des Schafts 50 andererseits sind so aneinander angepasst, dass die Knaggen 27 in den L-förmigen Nuten 57 bewegt werden können.

Ferner sind am proximalen Ende 51 des Schafts 50 zwei Ausnehmungen 75 vorgesehen, die einander bezogen auf die Längsachse 58 des proximalen Endes 51 des Schafts 50 gegenüberliegen. Die Anordnung und die Querschnitte der Ausnehmungen 75 am proximalen Ende 51 des Schafts 50 sind an die Anordnung und die Querschnitte der Riegel 72 am distalen Ende 22 der Handhabungseinrichtung 20 angepasst, so dass die Riegel 72 am distalen Ende 22 der Handhabungseinrichtung 20 in die Ausnehmungen 75 am proximalen Ende 51 des Schafts 50 eingreifen können, wenn die Knaggen 27 (vgl. Figur 3) in den von den axialen Abschnitten abgewandten Enden der umfänglichen Abschnitte der L-förmigen Nuten 57 angeordnet sind.

Ferner ist am proximalen Ende 51 des Schafts 50 eine Tülle 82 angeordnet, durch die eine Übertragungseinrichtung 81 in Form eines Drahts oder eines Seils aus dem proximalen Ende 51 des Schafts 50 austritt. Die Übertragungseinrichtung 81 weist an ihrem proximalen Ende eine in Figur 4 lediglich schematisch angedeutete Übertragungskupplungseinrichtung 85 zum mittelbaren oder unmittelbaren mechanischen Koppeln der Übertragungseinrichtung 81 mit einer Betätigungseinrichtung an einer Handhabungseinrichtung auf. Insbesondere ist die Übertragungskupplungseinrichtung 85 zur mechanischen Kopplung mit einem schwenkbaren oder entlang eines Wegs verschiebbaren Griffteil 23 (vgl. Figur 1) an einer Handhabungseinrichtung vorgesehen. Die Übertragungseinrichtung 81 koppelt dann das Griffteil 23 derart mit einem Werkzeug 14 am distalen Ende 52 des Schafts 50 (vgl. Figur 1), dass eine Bewegung des Griffteils 23 beispielsweise mit einer Bewegung von Maulteilen des Werkzeugs 14 einher geht.

Figur 5 zeigt eine weitere schematische Darstellung des distalen Endes 22 der Handhabungseinrichtung 20 und des proximalen Endes 51 des Schafts 50 aus den Figuren 1 bis 4. Die Zeichenebene der Figur 5 ist parallel zu den Zeichenebenen der Figuren 1 und 2 und zur Längsachse 58. Das distale Ende 22 der Handhabungseinrichtung 20 und das proximale Ende 51 des Schafts 50 sind ähnlich wie in Figur 2 voneinander beabstandet dargestellt. Im Unterschied zu Figur 2 sind jedoch keine von außen nicht sichtbaren Merkmale dargestellt. Stattdessen sind im Unterschied zu Figur 3 in Figur 5 die Riegel 72 und der Ring 73 dargestellt, die oben anhand der Figur 3 beschrieben sind.

Figur 6 zeigt eine schematische Schnittdarstellung des distalen Endes 22 der Handhabungseinrichtung 20 aus den Figuren 3 und 5 und des proximalen Endes 51 des Schafts 50 aus den Figuren 4 und 5. Die Schnittebene der Figur 6 ist senkrecht zu den Zeichenebenen der Figuren 1, 2 und 5 und parallel zur Längsachse 58.

In dem in Figur 6 dargestellten Schnitt ist erkennbar, dass die Knaggen 27 durch Stifte, die in die die Ausnehmung 25 umschließende Wand eingesetzt sind, gebildet sind. Ferner ist in Figur 6 erkennbar, dass die beiden Übertragungskupplungseinrichtungen 40 in zwei separaten und parallelen Führungskanälen 24 angeordnet sind.

Ferner sind in Figur 6 einige Merkmale des proximalen Endes 51 des Schafts 50 dargestellt, die in den Figuren 1 bis 5 nicht dargestellt sind. Im proximalen Ende 51 des Schafts 50 ist jede Übertragungskupplungseinrichtung 60 in einem separaten Führungskanal 56 angeordnet und geführt. Jede Übertragungskupplungseinrichtung 60 am proximalen Ende 51 des Schafts 50 umfasst an ihrem proximalen Ende einen konkaven Bereich bzw. eine Ausnehmung 64 mit einer Gestalt, die zur Kugel 46 am distalen Ende einer Übertragungskupplungseinrichtung am distalen Ende 22 der Handhabungseinrichtung 20 korrespondiert. Am distalen Ende weist jede Übertragungskupplungseinrichtung 60 im proximalen Ende 51 des Schafts 50 eine mechanische Verbindung 66 zu einem zugeordneten Zugseil 68 auf.

Die Querschnitte von distalen Abschnitten 65 der Übertragungskupplungseinrichtungen 60 und der Führungskanäle 56 am proximalen Ende 51 des Schafts 50 sind insbesondere kreisförmig. Proximale Abschnitte 63 der Übertragungskupplungseinrichtungen 60 können jeweils einen Querschnitt aufweisen, dessen Rand sich aus zwei gegenüberliegenden Kreisbögen und zwei gegenüberliegenden Geraden zusammensetzt. Insbesondere ist jede Übertragungskupplungseinrichtung aus einem proximalen Abschnitt 63 mit dem beschriebenen nicht-kreisförmigen Querschnitt und einem distalen Abschnitt 65 mit kreisförmigem Querschnitt zusammengesetzt, die beispielsweise miteinander verschraubt sind.

Die Figuren 7 bis 9 zeigen Darstellungen des proximalen Abschnitts 63 einer Übertragungskupplungseinrichtung 60 am proximalen Ende 51 eines Schafts 50 (vgl. Figur 6). Figur 7 zeigt eine schematische Schnittdarstellung, wobei die Schnittebene parallel zu den Zeichenebenen der Figuren 2 und 5, senkrecht zur Zeichenebene der Figur 6 und parallel zur Längsachse 58 ist. Figur 8 zeigt eine Draufsicht, wobei die Zeichenebene senkrecht zu den Zeichenebenen der Figuren 2 und 5, parallel zur Zeichenebene der Figur 6 und parallel zur Längsachse 58 ist. Figur 9 zeigt eine axonometrische Darstellung.

In der Zusammenschau der Figuren 7 bis 9 ist die Gestalt des proximalen Endes des proximalen Abschnitts 63 einer Übertragungskupplungseinrichtung 60 am proximalen Ende 51 des Schafts 50 (vgl. Figur 6) erkennbar. Die Ausnehmung 64 wird im Wesentlichen durch eine Bohrung gebildet, die einen kreisförmigen Querschnitt aufweist und quer zur Längsachse 58 (vgl. Figuren 3 bis 6) verläuft. Proximal dieser Bohrung ist ein im Wesentlichen trichterartiger Bereich 67 vorgesehen, dessen Funktion und Wirkung unten mit Bezug auf Figur 12 dargestellt ist.

Figuren 10 und 11 zeigen schematische Darstellungen der Kugeln 46 an den distalen Enden der Übertragungskupplungseinrichtungen 40 in der Handhabungseinrichtung 20 (vgl. Figuren 2, 3, 6) und der Übertragungskupplungseinrichtung 60 am proximalen Ende 51 des Schafts 50 (vgl. Figuren 4 und 6 bis 9). Die Zeichenebenen der Figuren 10 und 11 sind senkrecht zu den Zeichenebenen der Figuren 2 und 5 bis 8 und senkrecht zur Längsachse 58.

Figur 10 zeigt eine Situation bzw. Konfiguration, bei der das distale Ende 22 der Handhabungseinrichtung 20 und das proximale Ende 51 des Schafts 50 (vgl. Figuren 2 bis 6) ganz zusammengeführt, jedoch nicht miteinander mechanisch verbunden sind. Um zu der in Figur 10 gezeigten Konfiguration zu gelangen, werden die Knaggen 27 am distalen Ende 22 der Handhabungseinrichtung 20 (vgl. Figur 3) durch die axialen Abschnitte bis zu den umfänglichen Abschnitten, jedoch noch nicht entlang den umfänglichen Abschnitten der L-förmigen Nuten 57 am proximalen Ende 51 des Schafts 50 bewegt. Die Kugeln 46 an den distalen Enden der Übertragungskupplungseinrichtungen 40 der Handhabungseinrichtung 20 sind noch nicht in den Ausnehmungen 64 der Übertragungskupplungseinrichtungen 60 am proximalen Ende 51 des Schafts 50 angeordnet.

Gekrümmte Pfeile 94 deuten eine Rotationsbewegung der Handhabungseinrichtung 20 (vgl. Figur 2) zusammen mit den Kugeln 46 an den distalen Enden der Übertragungskupplungseinrichtungen 40 relativ zum proximalen Ende 51 des Schafts 50 und den darin geführten Übertragungskupplungseinrichtungen 60 an. Bei dieser Rotationsbewegung 94 werden die Knaggen 27 am distalen Ende 22 der Handhabungseinrichtung 20 in den umfänglichen Abschnitten der L-förmigen Nuten 57 bewegt. Außerdem können dabei die Kugeln 46 in die Ausnehmungen 64 bewegt werden.

Figur 11 zeigt eine Konfiguration, bei der die Kugeln 46 an den distalen Enden der Übertragungskupplungseinrichtungen 40 der Handhabungseinrichtung 20 in den Ausnehmungen 64 der Übertragungskupplungseinrichtungen 60 am proximalen Ende 51 des Schafts 50 angeordnet sind. Dadurch sind je eine Übertragungskupplungseinrichtung 40 in der Handhabungseinrichtung 20 und eine Übertragungskupplungseinrichtung 60 am proximalen Ende 51 des Schafts 50 formschlüssig mechanisch gekoppelt: Eine Bewegung einer Übertragungskupplungseinrichtung 40 der Handhabungseinrichtung 20 parallel zur Längsachse 58 geht mit einer entsprechenden Bewegung der Übertragungskupplungseinrichtungen 60 am proximalen Ende 51 des Schafts 50 einher, und Kräfte können übertragen werden.

Bei der in Figur 11 gezeigten Konfiguration sind die Knaggen 27 am distalen Ende 22 der Handhabungseinrichtung 20 in den von den axialen Abschnitten beabstandeten Enden der umfänglichen Abschnitte der L-förmigen Nuten 57 am proximalen Ende 51 des Schafts 50 angeordnet. Dadurch sind Handhabungseinrichtung 20 und Schaft 50 mechanisch starr miteinander verbunden. In dieser Konfiguration können die Riegel 72 am distalen Ende 22 der Handhabungseinrichtung 20 (vgl. Figuren 3 und 5) in die Ausnehmungen 75 am proximalen Ende 51 des Schafts 50 (vgl. Figur 4) eingreifen und die mechanische Verbindung von Handhabungseinrichtung 20 und Schaft 50 verriegeln.

Wenn die Riegel 72 mittels des Sicherungsrings 73 (vgl. Figur 3; insbesondere gegen die Kraft von Federn oder anderen elastischen Elementen) nach proximal verschoben und damit aus den Ausnehmungen 75 am proximalen Ende 51 des Schafts 50 herausgezogen sind, ist die mechanische Verbindung von Schaft 50 und Handhabungseinrichtung 20 entriegelt. In diesem entriegelten Zustand kann eine durch Pfeile 96 in Figur 11 angedeutete Rotationsbewegung der Handhabungseinrichtung 20 zusammen mit den Kugeln 46 an den distalen Enden der Übertragungskupplungseinrichtungen 40 relativ zum proximalen Ende 51 des Schafts 50 ausgeführt werden. Bei dieser Rotationsbewegung 96 werden die Kugeln 46 aus den Ausnehmungen 64 in den Übertragungskupplungseinrichtungen 60 am proximalen Ende 51 des Schafts 50 herausbewegt. Dabei werden gleichzeitig die Knaggen 27 am distalen Ende 22 der Handhabungseinrichtung 20 durch die umfänglichen Abschnitte bis zu den axialen Abschnitten der L-förmigen Nuten 57 am proximalen Ende 51 des Schafts 50 bewegt (vgl. Figuren 3 bis 5). Dadurch wird die mechanische Kopplung zwischen den mechanischen Übertragungskupplungseinrichtungen 40, 60 der Handhabungseinrichtung 20 und des Schafts 50 getrennt. Danach können die Handhabungseinrichtung 20 und das proximale Ende 51 des Schafts 50 durch eine Relativbewegung parallel zur Längsachse 58 voneinander getrennt werden. Dabei werden die Knaggen 27 am distalen Ende 22 der Handhabungseinrichtung 20 durch die axialen Abschnitte der L-förmigen Nuten 57 am proximalen Ende 51 des Schafts 50 hindurchbewegt.

Jede Übertragungskupplungseinrichtung 40 der Handhabungseinrichtung 20 ist unabhängig von der zugeordneten Übertragungskupplungseinrichtung 60 am proximalen Ende 51 des Schafts 50 parallel zur Längsachse 58 bewegbar. Jedoch kann nur in einer relativen Positionierung beider Übertragungskupplungseinrichtungen 40, 60 die Kugel 46 am distalen Ende der Übertragungskupplungseinrichtung 40 der Handhabungseinrichtung 20 unmittelbar in die Ausnehmung 64 am proximalen Ende der Übertragungskupplungseinrichtung 60 des Schafts 50 aufgenommen werden, wie dies in Figur 10 durch die Pfeile 94 angedeutet ist. Wie nachfolgend anhand der Figur 12 beschrieben ist, reicht jedoch ein vollständiges Durchfahren aller möglichen Positionen der Kugeln 46 (insbesondere durch Betätigung der Betätigungseinrichtung 30 - vgl. Figur 2) aus, um eine Aufnahme der Kugeln 46 in den Ausnehmungen 64 und damit eine mechanische Kopplung der jeweils zugeordneten Übertragungskupplungseinrichtungen 40, 60 zu bewirken.

Figur 12 zeigt eine schematische Schnittdarstellung einer Übertragungskupplungseinrichtung 40 in einem Führungskanal 24 der Handhabungseinrichtung 20 und der zugeordneten Übertragungskupplungseinrichtung 60 im Führungskanal 56 am proximalen Ende 51 des Schafts 50. Die Schnittebene der Figur 12 ist parallel zu den Zeichenebenen der Figuren 1, 2, 5, 7, senkrecht zu den Zeichenebenen der Figuren, 6, 8, 10, 11 und parallel zur Längsachse 58. Ein Pfeil 98 deutet eine Bewegung an, die von einer Position der Kugel 46 proximal des proximalen Endes der Übertragungskupplungseinrichtung 60 des Schafts 50 ausgeht, und bei der die Übertragungskupplungseinrichtung 40 der Handhabungseinrichtung 20 relativ zur Übertragungskupplungseinrichtung 60 des Schafts 50 nach distal verschoben wird. Der oben anhand der Figuren 7 bis 9 dargestellte trichterartige Bereich 67 bewirkt ähnlich wie eine Rampe oder ein Keil eine Auslenkung der Kugel 46 senkrecht zur Längsachse 58 bis zu der in Figur 12 gezeigten Position, bei der der elastische Abschnitt 44 der Übertragungskupplungseinrichtung 40 der Handhabungseinrichtung 20 elastisch verformt ist. Wenn die Übertragungskupplungseinrichtung 40 der Handhabungseinrichtung 20 von der in Figur 12 dargestellten Position aus relativ zur Übertragungskupplungseinrichtung 60 des Schafts 50 noch weiter nach distal verschoben wird, gelangt die Kugel 46 wie durch den Pfeil 98 angedeutet, in die Ausnehmung 64. In dieser nicht dargestellten Position, liegt die auch oben anhand der Figur 11 dargestellte mechanische Kopplung der zugeordneten Übertragungskupplungseinrichtungen 40, 60 vor.

### Bezugszeichen

- 10: Medizinisches Instrument
- 11: proximales Ende des medizinischen Instruments 10
- 12: distales Ende des medizinischen Instruments 10
- 14: Werkzeug am distalen Ende 12 des medizinischen Instruments 10
- 20: Handhabungseinrichtung
- 22: distales Ende der Handhabungseinrichtung 20
- 23: bewegbares Griffteil
- 24: Führungskanal für Übertragungskupplungseinrichtung 40 in der Handhabungseinrichtung 20
- 25: Ausnehmung am distalen Ende 22 der Handhabungseinrichtung 20
- 27: Knagge an der Handhabungseinrichtung 20
- 30: Betätigungseinrichtung an der Handhabungseinrichtung 20
- 32: mit der Betätigungseinrichtung 30 starr verbundene Kurbel
- 34: Pleuel zwischen Kurbel 32 und erster Übertragungskupplungseinrichtung 40
- 38: Schwenkachse der Betätigungseinrichtung 30
- 40: Übertragungskupplungseinrichtung an der Handhabungseinrichtung 20
- 42: im Führungskanal 24 geführter Abschnitt der Übertragungskupplungseinrichtung 40
- 44: elastischer Abschnitt an der Übertragungskupplungseinrichtung 40
- 46: Kugel am distalen Ende der Übertragungskupplungseinrichtung 40
- 50: Schaft des medizinischen Instruments 10
- 51: proximales Ende des Schafts 50
- 52: distales Ende des Schafts 50
- 53: ungesteuert flexibler Abschnitt des Schafts 50
- 54: gesteuert krümmbarer Abschnitt des Schafts 50
- 55: konvexer Bereich am proximalen Ende 51 des Schafts 50
- 56: Führungskanal für Übertragungskupplungseinrichtung 60 im proximalen Ende 51 des Schafts 50
- 57: L-förmige Nut am proximalen Ende 51 des Schafts 50 für Knagge 27 an der Handhabungseinrichtung 20
- 58: Längsachse des proximalen Endes 51 des Schafts 50
- 59: Ausnehmung an proximalem Ende 51 des Schafts 50 für Riegel 72 an Handhabungseinrichtung 20
- 60: Übertragungskupplungseinrichtung am proximalen Ende 51 des Schafts 50
- 63: proximaler Abschnitt der Übertragungskupplungseinrichtung 60
- 64: zur Kugel 46 korrespondierende Ausnehmung an der Übertragungskupplungseinrichtung 60
- 65: distaler Abschnitt der Übertragungskupplungseinrichtung 60
- 66: mechanische Verbindung der Übertragungskupplungseinrichtung 60 mit dem Zugseil 68
- 67: trichterartiger Bereich am proximalen Abschnitt 63 der Übertragungskupplungseinrichtung 60
- 68: Zugseil im Schaft 50 zum Steuern des gesteuert flexiblen Abschnitts 54
- 72: Riegel an der Handhabungseinrichtung 20
- 73: Ring an der Handhabungseinrichtung 20
- 75: Ausnehmung an proximalem Ende 51 des Schafts 50 für Riegel 72 an Handhabungseinrichtung 20
- 81: weitere Übertragungseinrichtung
- 82: Tülle
- 85: weitere Übertragungskupplungseinrichtung
- 94: Rotationsbewegung zum Verbinden
- 96: Rotationsbewegung zum Trennen
- 98: Koppeln der Relativbewegung der Übertragungskupplungseinrichtungen 40, 60

## Patentansprüche

1. Schaft (50) für ein medizinisches Instrument (10), mit:
einem Werkzeug (14) oder einer Kupplung für ein Werkzeug (14);
einem gesteuert krümmbaren Abschnitt (54) oder einem Gelenk;
einem ungesteuert flexiblen Abschnitt (53); **gekennzeichnet durch** eine Schaftkupplungseinrichtung (57) zur lösbaren mechanischen Verbindung des Schafts (50) mit einer Handhabungseinrichtung (20); und eine Übertragungskupplungseinrichtung (60) am proximalen Ende (51) des Schafts (50) zum lösbaren mechanischen Koppeln mit einer korrespondierenden Übertragungskupplungseinrichtung (40) an einer Handhabungseinrichtung (20),
wobei die Schaftkupplungseinrichtung (57) und die Übertragungskupplungseinrichtung (60) so angeordnet und ausgebildet sind, dass eine mechanische Verbindung zwischen der Schaftkupplungseinrichtung (57) und einer korrespondierenden Kupplungseinrichtung (27) an einer Handhabungseinrichtung (20) und eine mechanische Kopplung der Übertragungskupplungseinrichtung (60) mit einer korrespondierenden Übertragungskupplungseinrichtung (40) an der Handhabungseinrichtung (20) mittels einer Rotation des Schafts (50) relativ zur Handhabungseinrichtung (20) lösbar sind,
wobei die Übertragungskupplungseinrichtung (60) für eine Rastverbindung mit einer korrespondierenden Übertragungskupplungseinrichtung (40) an einer Handhabungseinrichtung (20) ausgebildet ist.

2. Schaft (50) nach dem vorangehenden Anspruch, ferner mit:
einer Übertragungseinrichtung (68) zum Übertragen zumindest entweder einer Kraft oder eines Drehmoments zwischen dem proximalen Ende (51) des Schafts (50) und dem gesteuert krümmbaren Abschnitt (54) oder dem Gelenk.

3. Schaft (50) nach dem vorangehenden Anspruch, bei dem
die Übertragungseinrichtung ein Zugseil (68) oder einen Zugdraht umfasst.

4. Schaft (50) nach einem der vorangehenden Ansprüche, bei dem
die Übertragungskupplungseinrichtung (60) an ihrem proximalen Ende einen konkaven Abschnitt (64) aufweist.

5. Schaft (50) nach einem der vorangehenden Ansprüche, bei dem
am proximalen Ende (51) des Schafts (50) mehrere Übertragungskupplungseinrichtungen (60) zur Steuerung des gesteuert krümmbaren Abschnitts vorgesehen sind.

6. Schaft (50) nach einem der vorangehenden Ansprüche, ferner mit:
einer weiteren Übertragungseinrichtung (81) zum Übertragen zumindest entweder einer Kraft oder eines Drehmoments zwischen dem proximalen Ende (51) des Schafts (50) und einem Werkzeug (14) am distalen Ende (52) des Schafts (50);
einer weiteren Übertragungskupplungseinrichtung (85) zum mechanischen Koppeln der Übertragungseinrichtung (81) mit einer korrespondierenden Übertragungskupplungseinrichtung (85) an einer Handhabungseinrichtung (20),
wobei die weitere Übertragungseinrichtung (85) außerhalb der Schaftkupplungseinrichtung (57) angeordnet ist.

7. Handhabungseinrichtung (20) für ein medizinisches Instrument (10), mit:
einer Schaftkupplungseinrichtung (27) zur lösbaren mechanischen Verbindung der Handhabungseinrichtung (20) mit einem Schaft (50) mit einem gesteuert krümmbaren Abschnitt (54) oder einem Gelenk und mit einem ungesteuert flexiblen Abschnitt (53);
einer ersten Betätigungseinrichtung (23) zum Steuern eines Werkzeugs (14) am distalen Ende (52) des Schafts (50);
einer zweiten Betätigungseinrichtung (30) zum manuellen Steuern der Krümmung des gesteuert krümmbaren Abschnitts (54) oder des Gelenks des Schafts (50);
einer Übertragungskupplungseinrichtung (40) zum lösbaren mechanischen Koppeln der zweiten Betätigungseinrichtung (30) mit einer Übertragungseinrichtung (68) zum Übertragen zumindest entweder einer Kraft oder eines Drehmoments zu dem gesteuert krümmbaren Abschnitt (54) oder Gelenk des Schafts (50),
wobei die Schaftkupplungseinrichtung (27) und die Übertragungskupplungseinrichtung (40) so angeordnet und ausgebildet sind, dass eine mechanische Verbindung zwischen der Schafkupplungseinrichtung (27) und einer korrespondierenden Kupplungseinrichtung (57) an einem Schaft (50) und eine mechanische Kopplung der Übertragungskupplungseinrichtung (40) mit einer korrespondierenden Übertragungskupplungseinrichtung (60) an dem Schaft (50) mittels einer Rotation der Handhabungseinrichtung (20) relativ zum Schaft (50) lösbar sind,
wobei die Übertragungskupplungseinrichtung (40) für eine Rastverbindung mit einer korrespondierenden Übertragungskupplungseinrichtung (60) an einem mit der Handhabungseinrichtung (20) verbundenen Schaft (50) ausgebildet ist.

8. Handhabungseinrichtung (20) nach dem vorangehenden Anspruch, bei der
die Übertragungskupplungseinrichtung (40) an ihrem distalen Ende einen konvexen Abschnitt (46) aufweist.

9. Schaft (50) nach einem der Ansprüche 1 bis 6 oder Handhabungseinrichtung (20) nach einem der Ansprüche 7 und 8, wobei mehrere Übertragungskupplungseinrichtungen (40; 60) symmetrisch bezüglich der Längsachse (58) des Schafts (50) angeordnet sind.

10. Medizinisches Instrument (10) mit einem Schaft (50) nach einem der Ansprüche 1 bis 6 und 9 und einer Handhabungseinrichtung (20) nach einem der Ansprüche 7 bis 9.

## Claims

1. Shank (50) for a medical instrument (10), having:
a tool (14) or a coupling for a tool (14);
a controlled curvable portion (54) or a hinge;
an uncontrolled flexible portion (53);
**characterized by** a shank coupling device (57) for the releasable mechanical connection of the shank (50) to a maneuvering device (20);
and a transmission coupling device (60) at the proximal end (51) of the shank (50), for releasable mechanical coupling to a corresponding transmission coupling device (40) on a maneuvering device (20),
wherein the shank coupling device (57) and the transmission coupling device (60) are arranged and designed in such a way that a mechanical connection between the shank coupling device (57) and a corresponding coupling device (27) on a maneuvering device (20) and a mechanical coupling of the transmission coupling device (60) to a corresponding transmission coupling device (40) on the maneuvering device (20) can be released by means of a rotation of the shank (50) relative to the maneuvering device (20),
wherein the transmission coupling device (60) is designed for a latching connection to a corresponding transmission coupling device (40) on a maneuvering device (20).

2. Shank (50) according to the preceding claim, also having:
a transmission device (68) for transmitting at least either a force or a torque between the proximal end (51) of the shank (50) and the controlled curvable portion (54) or the hinge.

3. Shank (50) according to the preceding claim, in which
the transmission device comprises a tensioning cable (68) or a tensioning wire.

4. Shank (50) according to one of the preceding claims, in which
the transmission coupling device (60) has, at its proximal end, a concave portion (64).

5. Shank (50) according to one of the preceding claims, in which
several transmission coupling devices (60) for controlling the controlled curvable portion are provided at the proximal end (51) of the shank (50).

6. Shank (50) according to one of the preceding claims, also having:
a further transmission device (81) for transmitting at least either a force or a torque between the proximal end (51) of the shank (50) and a tool (14) at the distal end (52) of the shank (50);
a further transmission coupling device (85) for the mechanical coupling of the transmission device (81) to a corresponding transmission coupling device (85) on a maneuvering device (20),
wherein the further transmission device (85) is arranged outside the shank coupling device (57).

7. Maneuvering device (20) for a medical instrument (10), having:
a shank coupling device (27) for the releasable mechanical connection of the maneuvering device (20) to a shank (50) with a controlled curvable portion (54) or a hinge and with an uncontrolled flexible portion (53);
a first actuating device (23) for controlling a tool (14) at the distal end (52) of the shank (50);
a second actuating device (30) for manually controlling the curvature of the controlled curvable portion (54) or hinge of the shank (50);
a transmission coupling device (40) for the releasable mechanical coupling of the second actuating device (30) to a transmission device (68) for transmitting at least either a force or a torque to the controlled curvable portion (54) or hinge of the shank (50),
wherein the shank coupling device (27) and the transmission coupling device (40) are arranged and designed in such a way that a mechanical connection between the shank coupling device (27) and a corresponding coupling device (57) on a shank (50) and a mechanical coupling of the transmission coupling device (40) to a corresponding transmission coupling device (60) on the shank (50) can be released by means of a rotation of the maneuvering device (20) relative to the shank (50),
wherein the transmission coupling device (40) is designed for a latching connection to a corresponding transmission coupling device (60) on a shank (50) connected to the maneuvering device (20).

8. Maneuvering device (20) according to the preceding claim, in which
the transmission coupling device (40) has, at its distal end, a convex portion (46).

9. Shank (50) according to one of Claims 1 to 6 or maneuvering device (20) according to either of Claims 7 and 8, wherein several transmission coupling devices (40; 60) are arranged symmetrically with respect to the longitudinal axis (58) of the shank (50).

10. Medical instrument (10) having a shank (50) according to one of Claims 1 to 6 and 9 and having a maneuvering device (20) according to one of Claims 7 to 9.

## Revendications

1. Tige (50) pour un instrument médical (10), avec:
un outil (14) ou un raccord pour un outil (14);
une partie pouvant être courbée de façon ciblée (54) ou une articulation;
une partie pouvant être courbée de façon non ciblée (53);
**caractérisée par** un dispositif de couplage de tige (57) pour le raccordement mécanique séparable de la tige (50) avec un dispositif de manipulation (20); et
un dispositif de couplage de transmission (60) à l'extrémité proximale (51) de la tige (50) pour le couplage mécanique séparable avec un dispositif de couplage de transmission correspondant (40) sur un dispositif de manipulation (20),
dans laquelle le dispositif de couplage de tige (57) et le dispositif de couplage de transmission (60) sont disposés et configurés de telle manière qu'un raccordement mécanique entre le dispositif de couplage de tige (57) et un dispositif de couplage correspondant (27) sur un dispositif de manipulation (20) et un couplage mécanique du dispositif de couplage de transmission (60) avec un dispositif de couplage de transmission correspondant (40) sur le dispositif de manipulation (20) soient séparables au moyen d'une rotation de la tige (50) par rapport au dispositif de manipulation (20),
dans laquelle le dispositif de couplage de transmission (60) est réalisé en vue d'une liaison par encliquetage avec un dispositif de couplage de transmission correspondant (40) sur un dispositif de manipulation (20).

2. Tige (50) selon la revendication précédente, avec en outre un dispositif de transmission (68) pour transmettre au moins soit une force soit un couple entre l'extrémité proximale (51) de la tige (50) et la partie pouvant être courbée de façon ciblée (54) ou l'articulation.

3. Tige (50) selon la revendication précédente, dans laquelle le dispositif de couplage de transmission comprend un câble de traction (68) ou un fil de traction.

4. Tige (50) selon l'une quelconque des revendications précédentes, dans laquelle le dispositif de couplage de transmission (60) présente à son extrémité proximale une partie concave (64).

5. Tige (50) selon l'une quelconque des revendications précédentes, dans laquelle il est prévu à l'extrémité proximale (51) de la tige (50) plusieurs dispositifs de couplage de transmission (60) pour la commande de la partie pouvant être courbée de façon ciblée.

6. Tige (50) selon l'une quelconque des revendications précédentes, avec en outre:
un autre dispositif de transmission (81) pour transmettre au moins soit une force soit un couple entre l'extrémité proximale (51) de la tige (50) et un outil (14) à l'extrémité distale (52) de la tige (50);
un autre dispositif de couplage de transmission (85) pour coupler mécaniquement le dispositif de transmission (81) à un dispositif de couplage de transmission correspondant (85) sur un dispositif de manipulation (20),
dans laquelle l'autre dispositif de transmission (85) est disposé à l'extérieur du dispositif de couplage de tige (57).

7. Dispositif de manipulation (20) pour un instrument médical (10), avec:
un dispositif de couplage de tige (27) pour le raccordement mécanique séparable du dispositif de manipulation (20) avec une tige (50) avec une partie pouvant être courbée de façon ciblée (54) ou une articulation et avec une partie pouvant être courbée de façon non ciblée (53);
un premier dispositif d'actionnement (23) pour commander un outil (14) à l'extrémité distale (52) de la tige (50);
un deuxième dispositif d'actionnement (30) pour commander manuellement la courbure de la partie pouvant être courbée de façon ciblée (54) ou l'articulation de la tige (50);
un dispositif de couplage de transmission (40) pour le couplage mécanique séparable du deuxième dispositif d'actionnement (30) avec un dispositif de transmission (68) pour transmettre au moins soit une force soit un couple à la partie pouvant être courbée de façon ciblée (54) ou à l'articulation de la tige (50);
dans lequel le dispositif de couplage de tige (27) et le dispositif de couplage de transmission (40) sont disposés et configurés de telle manière qu'un raccordement mécanique entre le dispositif de couplage de tige (27) et un dispositif de couplage correspondant (57) sur une tige (50) et un couplage mécanique du dispositif de couplage de transmission (40) avec un dispositif de couplage de transmission correspondant (60) sur la tige (50) soient séparables au moyen d'une rotation du dispositif de manipulation (20) par rapport à la tige (50),
dans lequel le dispositif de couplage de transmission (40) est réalisé en vue d'une liaison par encliquetage avec un dispositif de couplage de transmission correspondant (60) sur une tige (50) reliée au dispositif de manipulation (20).

8. Dispositif de manipulation (20) selon la revendication précédente, dans lequel le dispositif de couplage de transmission (40) présente à son extrémité distale une partie convexe (46).

9. Tige (50) selon l'une quelconque des revendications 1 à 6 ou dispositif de manipulation (20) selon une des revendications 7 et 8, dans laquelle / lequel plusieurs dispositifs de couplage de transmission (40; 60) sont disposés de façon symétrique par rapport à l'axe longitudinal (58) de la tige (50).

10. Instrument médical (10) avec une tige (50) selon l'une quelconque des revendications 1 à 6 et 9 et un dispositif de manipulation (20) selon l'une quelconque des revendications 7 à 9.
